# EUROPÄISCHE PATENTSCHRIFT

(11) **EP 1 521 722 B1**
(45) Veröffentlichungstag und Bekanntmachung des Hinweises auf die Patenterteilung: **05.04.2006**
(21) Anmeldenummer: 03738116.7
(22) Anmeldetag: 04.07.2003
(51) Int. Cl.: C01G 9/04, B01D 3/36, C07C 253/34

(54) **VERFAHREN ZUR ENTFERNUNG VON WASSER AUS EINER MISCHUNG, DIE WASSER UND ZINKCHLORID ENTH LT**
METHOD FOR REMOVING WATER FROM A MIXTURE CONTAINING WATER AND ZINC CHLORIDE
PROCEDE POUR ELIMINER DE L'EAU D'UN MELANGE QUI CONTIENT DE L'EAU ET DU CHLORURE DE ZINC

(30) Priorität: 10.07.2002 DE 10231296
(43) Veröffentlichungstag der Anmeldung: 13.04.2005
(73) Patentinhaber: BASF Aktiengesellschaft, 67056 Ludwigshafen (DE)
(72) Erfinder: JUNGKAMP, Tim, 2950 Kapellen (NL); SCHEIDEL, Jens, 69493 Hirschberg (DE); LUYKEN, Hermann, 67069 Ludwigshafen (DE); BARTSCH, Michael, 67433 Neustadt (DE); BAUMANN, Robert, 68159 Mannheim (DE); HADERLEIN, Gerd, 67269 Grünstadt (DE)
(86) Internationale Anmeldenummer: PCT/EP2003/007149
(87) Internationale Veröffentlichungsnummer: WO 2004/007371

(56) Entgegenhaltungen:
- EP-A- 0 268 448
- EP-A- 0 372 922
- WO-A-00/38813
- GB-A- 469 352
- US-A- 3 766 241

## Beschreibung

Die vorliegende Erfindung betrifft ein Verfahren zur Entfernung von Wasser aus einer Mischung, enthaltend Wasser und Zinkchlorid, dadurch gekennzeichnet, daß man
die besagte Mischung, enthaltend Wasser und Zinkchlorid, mit einem aprotischen, polaren Verdünnungsmittel versetzt,
dessen Siedepunkt im Falle der Nichtazeotrop-Bildung des genannten Verdünnungsmittels mit Wasser unter den Druckbedingungen der nachfolgend genannten Destillation höher ist als der Siedepunkt von Wasser und das an diesem Siedepunkt des Wassers flüssig vorliegt
oder
das ein Azeotrop oder Heteroazeotrop mit Wasser unter den Druck- und Temperaturbedingungen der nachfolgend genannten Destillation bildet,
und
die Mischung, enthaltend Wasser, Zinkchlorid und das Verdünnungsmittel, destilliert unter Abtrennung von Wasser oder des genannten Azetrops oder des genannten Heteroazeotrops von dieser Mischung und unter Erhalt einer wasserfreien Mischung, enthaltend Zinkchlorid und das besagte Verdünnungsmittel.

Wasserfreies Zinkchlorid oder wasserfreie Mischungen, die ein flüssiges Verdünnungsmittel und Zinkchlorid enthalten, stellen technisch wichtige Ausgangsverbindungen dar, die unter anderem Verwendung in der Galvanisiertechnik bei der Verzinkung von metallischen Materialien oder wegen der Lewis-Säure-Eigenschaften des Zinkchlorids als Katalysator oder Katalysatorbestandteil finden.

Nach solchen Verwendungen stellt sich bekanntermaßen das Problem, aus dem nach der Verwendung erhaltenen Gemisch das Zinkchlorid in einer solchen Form zurückzugewinnen, daß es einer technischen Verwendung erneut wieder zugeführt werden kann, üblicherweise in Form von wasserfreiem Zinkchlorid oder wasserfreien Mischungen, die ein flüssiges Verdünnungsmittel und Zinkchlorid enthalten.

Üblicherweise fallen bei dieser Rückgewinnung Mischungen an, die neben Zinkchlorid auch Wasser enthalten.

Die besondere Schwierigkeit besteht darin, aus solchen Mischungen wasserfreies Zinkchlorid oder wasserfreie Mischungen, die ein flüssiges Verdünnungsmittel und Zinkchlorid enthalten, zurückzugewinnen, wie beispielsweise aus Report of Investigations Nr. 9347, "Method for Recovering Anhydrous ZnCl₂ from Aqueous Solutions", B.R. Eichbaum, L.E. Schultze, United States Department of the Interior, Bureau of Mines, 1991, Seite 1-10, ("RI 9347") bekannt.

Bei der Einengung von wässrigen Zinkchlorid-Lösungen erhält man wegen der hohen Löslichkeit von Zinkchlorid in Wasser eine hochviskose Mutterlauge, die sehr schwierig zu filtrieren ist, um das ausgefallene Zinkchlorid von der Mutterlauge abzutrennen.

So ist aus RI 9347, Seite 4, Tabelle 1 bekannt, daß bei der Trocknung von Zinkchlorid bei 70°C / 60 Tage bzw. bei 150°C / 8 Tage neben Zinkchlorid unbekannte Nebenprodukte erhalten werden. Zudem sind die genannten Trocknungszeiten für eine Rückgewinnung von Zinkchlorid unwirtschaftlich lang.

Gemäß RI 9347, Seite 4, Tabelle 2 wird beim Versuch, Zinkchlorid durch Sprühtrocknen einer Zinkchlorid-Lösung bei 100°C unter Luft darzustellen, kein Pulver, sondern eine feuchte Aufschlämmung erhalten.

In der in RI 9347 auf Seite 2 zitierten Literatur, wie auch in RI 9347 selbst wird vorgeschlagen, Zinkchlorid aus einer wässrigen Lösung unter Ammoniak- oder Ammonium-chlorid-Zusatz als Zink-diamin-dichlorid-Komplex auszufällen und aus diesem Zinkchlorid freizusetzen.

Wie aus RI 9347 ersichtlich, fällt bei diesem Verfahren kein reiner Zink-diamin-dichlorid-Komplex, sondern ein mit verschiedenen Zink-oxy- oder Zink-hydroxy-Verbindungen verunreinigter Zink-diamin-dichlorid-Komplex an.

Zudem beschreibt RI 9347 auf Seite 8, rechte Spalte, daß die Freisetzung von Zinkchlorid aus dem Zink-diamin-dichlorid-Komplex bei Temperaturen bis zu 400°C nicht vollständig erfolgt; bei Temperaturen oberhalb von 400°C werden nachteiligerweise explosive Gasmischungen erhalten, die auf die Zersetzung des Ammoniaks zurückzuführen sind.

GB469352 offenbart Verfahren zum Entwässern (Trocknen) von Zinkchforidlösungen durch azeotrope Destillation unter Verwendung von Kerosin.

Der vorliegenden Erfindung lag die Aufgabe zugrunde, ein Verfahren bereitzustellen, das die Entfernung von Wasser aus einer Mischung, enthaltend Wasser und Zinkchlorid, auf technisch einfache und wirtschaftliche Weise ermöglicht.

Demgemäß wurde das eingangs definierte Verfahren gefunden.

Gemäß erfindungsgemäßem Verfahren wird Wasser aus einer Mischung, enthaltend Wasser und Zinkchlorid, entfernt.

Das Mengenverhältnis von Wasser zu Zinkchlorid in der Ausgangsmischung ist an sich nicht kritisch. Mit zunehmendem Verhältnis von Zinkchlorid zu Wasser nimmt die Viskosität der Mischung deutlich zu, so daß die Handhabung der Mischung zunehmend aufwendiger wird.

Als vorteilhaft hat sich ein Anteil des Zinkchlorids an der Gewichtssumme aus Zinkchlorid und Wasser im Bereich von mindestens 0,01 Gew.-%, vorzugsweise mindestens 0,1 Gew.-%, besonders bevorzugt mindestens 0,25 Gew.-%, insbesondere bevorzugt mindestens 0,5 Gew.-% erwiesen.

Als vorteilhaft hat sich ein Anteil des Zinkchlorids an der Gewichtssumme aus Zinkchlorid und Wasser im Bereich von höchstens 60 Gew.-%, vorzugsweise höchstens 35 Gew.-%, besonders bevorzugt höchstens 30 Gew.-% erwiesen.

Die Ausgangsmischung kann aus Zinkchlorid und Wasser bestehen.

Die Ausgangsmischung kann neben Zinkchlorid und Wasser weitere Bestandteile enthalten, wie ionische oder nichtionische, organische oder anorganische Verbindungen, insbesondere solche, die mit der Ausgangsmischung homogen einphasig mischbar oder in der Ausgangsmischung löslich sind.

In einer bevorzugten Ausführungsform kommt die Zugabe einer anorganischen oder organischen Säure in Betracht. Bevorzugt können solche Säuren eingesetzt werden, die unter den Destillationsbedingungen gemäß erfindungsgemäßem Verfahren einen Siedepunkt aufweisen, der niedriger ist als der Siedepunkt des aprotischen, polaren Verdünnungsmittels. Besonders bevorzugt kommen Halogenwasserstoffsäuren, wie HF, HCl, HBr, HJ, insbesondere HCl in Betracht.

Die Menge der Säure kann vorteilhaft so gewählt werden, dass der pH-Wert der Mischung, enthaltend Wasser und Zinkchlorid, kleiner als 7 ist.

Die Menge der Säure kann vorteilhaft so gewählt werden, dass der pH-Wert der Mischung, enthaltend Wasser und Zinkchlorid, größer-gleich 0, vorzugsweise größer-gleich 1 ist.

Solche Ausgangsmischungen können vorteilhaft erhalten werden durch Extraktion mit einem wasserhaltigen Extraktionsmittel, insbesondere mit Wasser, eines Reaktionsgemischs, das bei der Hydrocyanierung von Pentennitril in Gegenwart eines Katalysatorsystems, enthaltend Ni(0), einen oder mehrere phosphorhaltige Liganden und Zinkchlorid, zu Adipodinitril erhalten wurde.

Die Herstellung eines solchen Reaktionsgemischs ist an sich bekannt, beispielsweise aus US-A-4,705,881. Gemäß US-A-3,773,809 kann der Katalysator aus dem Reaktionsgemisch durch Extraktion, beispielsweise mit Cyclohexan, abgetrennt werden, wobei das Zinkchlorid in dem den überwiegenden Teil des Adipodinitrils enthaltenden Produktstrom verbleibt. Zur Gewinnung von reinem Adipodinitril kann das Zinkchlorid in an sich bekannter Weise aus einem solchen Produktstrom durch Reaktion mit Ammoniak entfernt werden, wie beispielsweise in US-A-3,766,241 beschrieben.

Vorteilhaft kann man die Extraktion unter Bedingungen durchführen, unter denen das Extraktionsmittel und das Reaktionsgemisch zweiphasig vorliegen.

Setzt man Wasser als Extraktionsmittel ein, so haben sich Temperaturen von mindestens 0°C, vorzugsweise mindestens 5°C, insbesondere mindestens 30°C als vorteilhaft erwiesen.

Setzt man Wasser als Extraktionsmittel ein, so haben sich Temperaturen von höchstens 200°C, vorzugsweise höchstens 100°C, insbesondere höchstens 50°C als vorteilhaft erwiesen.

Hierdurch stellen sich Drücke im Bereich von 10⁻³ bis 10 MPa, vorzugsweise von 10⁻² bis 1 MPa, insbesondere von 5*10⁻² bis 5*10⁻¹ MPa ein.

Die Phasentrennung kann in an sich bekannter Weise in für solche Zwecke beschriebenen Apparaturen erfolgen, wie sie beispielsweise aus: Ullmann's Encyclopedia of Industrial Chemistry, Vol. B3, 5. Ed., VCH Verlagsgesellschaft, Weinheim, 1988, Seite 6-14 bis 6-22 bekannt sind.

Die für die Phasentrennung optimalen Apparaturen und Verfahrensbedingungen lassen sich dabei leicht durch einige einfache Vorversuche ermitteln.

Erfindungsgemäß versetzt man die besagte Mischung, enthaltend Wasser und Zinkchlorid, mit einem aprotischen, polaren Verdünnungsmittel, dessen Siedepunkt unter den Druckbedingungen der nachfolgend genannten Destillation höher ist als der Siedepunkt von Wasser und das an diesem Siedepunkt des Wassers flüssig vorliegt. Die Zugabe von Wasser zu besagter Mischung kann vor der Destillation oder während der Destillation erfolgen.

Die Druckbedingungen für die nachfolgende Destillation sind an sich nicht kritisch. Als vorteilhaft haben sich Drücke von mindestens 10⁻⁴ MPa, vorzugsweise mindestens 10⁻³ MPa, insbesondere mindestens 5*10⁻³ MPa erwiesen.

Als vorteilhaft haben sich Drücke von höchstens 1 MPa, vorzugsweise höchstens 5*10⁻¹ MPa, insbesondere höchstens 1,5*10⁻¹ MPa erwiesen.

In Abhängigkeit von den Druckbedingungen und der Zusammensetzung des zu destillierenden Gemischs stellt sich dann die Destillationstemperatur ein.

Bei dieser Temperatur liegt das aprotische, polare Verdünnungsmittel erfindungsgemäß flüssig vor. Im Sinne der vorliegenden Erfindung wird unter dem Begriff aprotisches, polares Verdünnungsmittel sowohl ein einzelnes Verdünnungsmittel wie auch ein Gemisch solcher Verdünnungsmittel verstanden, wobei sich im Falle eines solchen Gemischs die erfindungsgemäßen, genannten physikalischen Eigenschaften auf dieses Gemisch beziehen.

Weiterhin weist das aprotische, polare Verdünnungsmittel erfindungsgemäß unter diesen Druck- und Temperaturbedingungen einen Siedepunkt auf, der im Falle der Nichtazeotrop-Bildung des Verdünnungsmittels mit Wasser höher als der von Wasser liegt, vorzugsweise um mindestens 5°C, insbesondere mindestens 20°C, und vorzugsweise höchstens 200°C, insbesondere höchstens 100°C.

Aprotische, polare organische und anorganische Verdünnungsmittel sind an sich bekannt, beispielsweise aus: Jerry March, Advanced Organic Chemistry, 2. Ed., Mc-Graw-Hill, International Student Edition, Hamburg, 8. Druck (1984), 1977, S. 331-336; Organikum, 2. Nachdruck der 15. Auflage, VEB Deutscher Verlag der Wissenschaften, Berlin, 1981, S. 226-227; Streitwieser/Heathcock, Organische Chemie, Verlag Chemie, Weinheim, 1980, S. 172.

In Betracht kommen beispielsweise Amide, insbesondere Dialkylamide, wie Dimethylformamid, Dimethylacetamid, N,N,-Dimethylethylenharnstoff (DMEU), N,N-Dimethylpropylenharnstoff (DMPU), Hexamethylenphosphorsäuretriamid (HMPT), Ketone, Schwefel-SauerstoffVerbindungen, wie Dimethylsulfoxid, Tetrahydrothiophen-1,1-dioxid, Nitroaromaten, wie Nitrobenzol, Nitroalkane, wie Nitromethan, Nitroethan, Ether, wie Diether des Diethylenglykols, beispielsweise Diethylenglykoldimethylether, Alkylencarbonate, wie Ethylencarbonat, Nitrile, wie Acetonitril, Propionitril, n-Butyronitril, n-Valeronitril, Cyanocyclopropan, Acrylnitril, Crotonitril, Allylcyanid, Pentennitrile.

Solche aprotischen, polaren Verdünnungsmittel können allein oder als Gemisch eingesetzt werden.

Solche aprotischen, polaren Verdünnungsmittel können weitere Verdünnungsmittel enthalten, vorzugsweise Aromaten, wie Benzol, Toluol, o-Xylol, m-Xylol, p-Xylol, Aliphaten, insbesondere Cycloaliphaten, wie Cyclohexan, Methylcyclohexan, oder deren Gemische.

In einer bevorzugten Ausführungsform kann man Verdünnungsmittel einsetzen, die mit Wasser ein Azeotrop oder Heteroazeotrop bilden. Die Menge an Verdünnungsmittel gegenüber der Menge an Wasser in dem Gemisch ist an sich nicht kritisch. Vorteilhaft sollte man mehr flüssiges Verdünnungsmittel einsetzen als den durch die Azeotrope abzudestillierenden Mengen entspricht, so daß überschüssiges Verdünnungsmittel als Sumpfprodukt verbleibt.

Setzt man ein Verdünnungsmittel ein, das mit Wasser kein Azeotrop bildet, so ist die Menge an Verdünnungsmittel gegenüber der Menge an Wasser in dem Gemisch an sich nicht kritisch.

Vorteilhaft kommen organische Verdünnungsmittel in Betracht, vorzugsweise solche mit mindestens einer Nitrilgruppe, insbesondere einer Nitrilgruppe.

In einer bevorzugten Ausführungsform kann man als Nitril ein aliphatisches, gesättigtes oder ein aliphatisches, olefinisch ungesättigtes Nitril einsetzen. Dabei kommen insbesondere Nitrile mit 3, 4, 5, 6, 7, 8, 9, 10, insbesondere 4 Kohlenstoffatomen, gerechnet ohne die Nitrilgruppen, vorzugsweise Nitrilgruppe, in Betracht.

In einer besonders bevorzugten Ausführungsform kann man als Verdünnungsmittel ein aliphatisches, olefinisch ungesättigtes Mononitril, ausgewählt aus der Gruppe bestehend aus 2-cis-Pentennitril, 2-trans-Pentennitril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische einsetzen.

2-cis-Pentennitril, 2-trans-Pentenniril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische sind bekannt und können nach an sich bekannten Verfahren, wie durch Hydrocyanierung von Butadien in Gegenwart von Katalysatoren, erhalten werden, beispielsweise gemäß US-A-3,496,215 oder die linearen Pentennitrile durch Isomerisierung von 2-Methyl-3-butennitril gemäß WO 97/23446.

Als besonders vorteilhaft kommen dabei solche Gemische der genannten Pentennitrile in Betracht, die 2-cis-Pentennitril, 2-trans-Pentennitril oder deren Gemische im Gemisch mit 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische enthalten. In solchen Gemischen findet bei der nachfolgenden Destillation eine Abreicherung von 2-cis-Pentennitril, 2-trans-Pentennitril, E-2-Methyl-2-butennitril, Z-2-Methyl-2-butennitril, 2-Methyl-3-butennitril oder deren Gemische statt, da diese mit Wasser Azeotrope bilden, die niedriger sieden als die Azeotrope von 3-cis-Pentennitril, 3-trans-Pentennitril, 4-Pentennitril oder deren Gemische mit Wasser. Bei dieser Ausführungsform erhält man nach der Destillation ein Gemisch enthaltend 3-cis-Pentennitril, 3-trans-Pentennitril, 4-Pentennitril oder deren Gemische und wasserfreies Zinkchlorid als Produkt des erfindungsgemäßen Verfahrens.

Dieses Produkt kann vorteilhaft zur weiteren Hydrocyanierung in Gegenwart eines Katalysators zu Adipodinitril eingesetzt werden. Eine Abreicherung von 2-cis-Pentennitril, 2-trans-Pentennitril, E-2-Methyl-2-butennitril, Z-2-Methyl-2-butennitril, 2-Methyl-3-butennitril ist dabei insoweit vorteilhaft, als daß diese Verbindungen der genannten Hydrocyanierung erheblich schlechter zugängig sind als 3-cis-Pentennitril, 3-trans-Pentennitril, 4-Pentennitril oder deren Gemische.

Setzt man als Verdünnungsmittel 2-cis-Pentennitril, 2-trans-Pentenniril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische ein, so haben sich Mengenverhältnisse von Pentennitril zu Zinkchlorid von mindestens 0,5 mol/mol, vorzugsweise mindestens 5 mol/mol, besonders bevorzugt mindestens 15 mol/mol als vorteilhaft erwiesen.

Setzt man als Verdünnungsmittel 2-cis-Pentennitril, 2-trans-Pentenniril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische ein, so haben sich Mengenverhältnisse von Pentennitril zu Zinkchlorid von höchstens 10000 mol/mol, vorzugsweise höchstens 5000 mol/mol, besonders bevorzugt höchstens 2000 mol/mol als vorteilhaft erwiesen.

Erfindungsgemäß destilliert man die Mischung, enthaltend Wasser, Zinkchlorid und das Verdünnungsmittel, unter Abtrennung von Wasser von dieser Mischung und unter Erhalt einer wasserfreien Mischung, enthaltend Zinkchlorid und das besagte Verdünnungsmittel.

Im Falle von Pentennitril als Verdünnungsmittel kann man die Destillation vorteilhaft bei einem Druck von höchstens 200 kPa, vorzugsweise höchstens 100 kPa, insbesondere höchstens 50 kPa, besonders bevorzugt höchstens 20 kPa durchführen.

Im Falle von Pentennitril als Verdünnungsmittel kann man die Destillation vorteilhaft bei einem Druck von mindestens 1 kPa, vorzugsweise mindestens 5 kPa, besonders bevorzugt 10 kPa durchführen.

Die Destillation kann vorteilhaft durch einstufige Verdampfung, bevorzugt durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen, Füllkörperkolonnen, Kolonnen mit Seitenabzug oder Trennwandkolonnen.

Die Destillation kann diskontinuierlich erfolgen.

Die Destillation kann kontinuierlich erfolgen.

Im Sinne der vorliegenden Erfindung wird unter wasserfreiem Zinkchlorid ein solches mit einem Gehalt an Wasser, bezogen auf die Gewichtssumme aus Zinkchlorid und Wasser, von weniger als 500 Gew.-ppm, vorzugsweise weniger als 50 Gew.-ppm, und mindestens gleich 0 Gew.-ppm verstanden.

### Beispiele

Die in den Beispielen angegebenen Gew.-%- oder Gew.-ppm-Angaben beziehen sich, soweit nicht anders bezeichnet, auf das Gesamtgewicht der jeweiligen Mischung.

Der Gehalt an Zn oder Zinkchlorid wurde mittels Atomemissionsspektrometrie bestimmt.

Der Gehalt an Chlor wurde nach Methode Schoeniger bestimmt.

Die Konzentrationen an Wasser wurden durch Titration nach Karl-Fischer-Methode potentiometrisch bestimmt.

### Beispiel 1

In einer kontinuierlich betriebenen Vakuum-Destillationskolonne mit Metallgewebepackung (Typ CY, Fa. Sulzer Chemtech, Innendurchmesser ⌀ = 50 mm, Höhe 130 cm) mit einem Dünnschichtverdampfer als Wärmeüberträger am Kolonnensumpf, einem bei 30°C betriebenem Kondensator am Kopf und einem auf 0°C gekühlten Phasentrenngefäß im Rücklauf wurden 240 g/h einer Lösung von 30 Gew.-% Zinkchlorid in trans-3-Pentennitril mit einem Wassergehalt von 0.4 Gew.-% oberhalb der Gewebepackung in die Destillationskolonne dosiert. Bei einem Druck von p = 100 kPa(absolut) wurde bei 344 K als Destillat am Kondensator ein zweiphasiges Gemisch erhalten. Die obere, im wesentlichen aus trans-3-Pentennitril bestehende Phase wurde kontinuierlich auf den Kolonnenkopf zurückgefahren. Die untere Phase bestand im wesentlichen aus Wasser und wurde kontinuierlich aus dem Phasentrenngefäß abgepumpt. Über Sumpf wurde eine homogene Lösung von ZnCl₂ in trans-3-Pentennitril bei 348 K abgetrennt. Nach 17 h Laufzeit der Destillation war der Wasseranteil im Sumpfaustrag auf 76 Gew-ppm H₂O, nach 41 h auf 50 Gew-ppm abgereichert.

### Beispiel 2

4 kg des in Beispiel 1 erhaltenen Sumpfaustrages wurden mit 1 kg trans-3-Pentenniril und 500 g Wasser versetzt. Die homogene Mischung wurde mit einer Dosierrate von 206 g/h in die wie in Beispiel 1 betriebene Destillationskolonne dosiert.

Der Sumpfaustrag enthielt nach 24 h kontinuierlichen Betriebs 350 Gew-ppm Wasser, 16.9 Gew.-% Chlor berechnet als Cl und 15.5 Gew.-% Zn, jeweils bezogen auf Gesamtgewicht der Lösung; daraus leitet sich ein experimentell gefundenes Cl:Zn -Verhältnis von 2.01 ab.

Gaschromatographische Analyse durch Derivatisierung mit MSTFA (2,2,2-trifluoro-N-methyl-N-(trimethylsilyl)acetamid) zeigte keine nachweisbaren Mengen an Verseifungsprodukt 3-Pentensäure.

Analyse auf polymere Abbauprodukte durch Gelpermeationschromatographie ergab keine nachweisbaren Mengen an polymerem Produkt.

Die so erhaltene Zinkchloridlösung in 3-Pentennitril kann in der Hydrocyanierung von 3-Pentennitril in Gegenwart von Nickel(0)-Phosphitkatalysatoren eingesetzt werden und zeigt keinen Aktivitätsunterschied zu einer frisch aus 3-Pentennitril und wasserfreiem Zinkchlorid hergestellten Lösung.

## Patentansprüche

1. Verfahren zur Entfernung von Wasser aus einer Mischung, enthaltend Wasser und Zinkchlorid, **dadurch gekennzeichnet, daß** man
die besagte Mischung, enthaltend Wasser und Zinkchlorid, mit einem aprotischen, polaren Verdünnungsmittel versetzt,
dessen Siedepunkt im Falle der Nichtazeotrop-Bildung des genannten Verdünnungsmittels mit Wasser unter den Druckbedingungen der nachfolgend genannten Destillation höher ist als der Siedepunkt von Wasser und das an diesem Siedepunkt des Wassers flüssig vorliegt
oder
das ein Azeotrop oder Heteroazeotrop mit Wasser unter den Druck- und Temperaturbedingungen der nachfolgend genannten Destillation bildet,
und
die Mischung, enthaltend Wasser, Zinkchlorid und das Verdünnungsmittel, destilliert unter Abtrennung von Wasser oder des genannten Azetrops oder des genannten Heteroazeotrops von dieser Mischung und unter Erhalt einer wasserfreien Mischung, enthaltend Zinkchlorid und das besagte Verdünnungsmittel, wobei man als aprotisch, polares Verdünnungsmittel ein aliphatisches, olefinisch ungesättigtes Nitril ausgewählt aus der Gruppe bestehend aus 2-cis-Pentennitril, 2-trans-Pentennitril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische einsetzt.

2. Verfahren nach den Anspruch 1, wobei das Verdünnungsmittel unter den Destillationsbedingungen ein Azeotrop oder Heteroazeotrop mit Wasser ausbilden kann.

3. Verfahren nach den Ansprüchen 1 bis 2, wobei die Mischung, enthaltend Wasser und Zinkchlorid einen pH-Wert von kleiner als 7 aufweist.

4. Verfahren nach den Ansprüchen 1 bis 3, wobei die Mischung, enthaltend Wasser und Zinkchlorid einen pH-Wert im Bereich von 0 bis kleiner als 7 aufweist.

5. Verfahren nach den Ansprüchen 1 bis 4, wobei man die Mischung, enthaltend Wasser und Zinkchlorid, mit einer Säure versetzt.

6. Verfahren nach Anspruch 5, wobei man als Säure HCl einsetzt.

## Claims

1. A process for the removal of water from a mixture comprising water and zinc chloride, which comprises
adding to said mixture comprising water and zinc chloride an aprotic, polar diluent
whose boiling point in the case where an azeotrope is not formed between said diluent and water under the pressure conditions of the distillation mentioned below is higher than the boiling point of water and which is in liquid form at this boiling point of water
or
which forms an azeotrope or heteroazeotrope with water under the pressure and temperature conditions of the distillation mentioned below,
and
distilling the mixture comprising water, zinc chloride and the diluent with removal of water or said azeotrope or said heteroazeotrope from this mixture, giving an anhydrous mixture comprising zinc chloride and said diluent, wherein the aprotic, polar diluent employed is an aliphatic, olefinically unsaturated nitrile selected from the group consisting of 2-cis-pentenenitrile, 2-trans-pentenenitrile, 3-cis-pentenenitrile, 3-trans-pentenenitrile, 4-pentenenitrile, E-2-methyl-2-butenenitrile, Z-2-methyl-2-butenenitrile, 2-methyl-3-butenenitrile or a mixture thereof.

2. The process according to claim 1, wherein the diluent is able to form an azeotrope or heteroazeotrope with water under the distillation conditions.

3. The process according to either of claims 1 and 2, wherein the mixture comprising water and zinc chloride has a pH of less than 7.

4. The process according to any one of claims 1 to 3, wherein the mixture comprising water and zinc chloride has a pH in the range from 0 to less than 7.

5. The process according to any one of claims 1 to 4, wherein an acid is added to the mixture comprising water and zinc chloride.

6. The process according to claim 5, wherein the acid employed is HCl.

## Revendications

1. Procédé pour l'élimination d'eau d'un mélange contenant de l'eau et du chlorure de zinc,
**caractérisé en ce que** :
l'on ajoute audit mélange contenant de l'eau et du chlorure de zinc un diluant aprotique polaire,
dont le point de fusion, dans le cas de la formation non azéotropique dudit diluant au moyen d'eau dans les conditions de pression de la distillation mentionnée ci-après, est supérieur au point de fusion de l'eau et qui a une forme liquide en ce point de fusion de l'eau
ou
qui forme un azéotrope ou un hétéroazéotrope avec de l'eau dans les conditions de pression et de température de la distillation mentionnée ci-après,
et
l'on distille le mélange, contenant de l'eau, du chlorure de zinc et le diluant, avec séparation d'eau ou de l'azéotrope susdit ou de l'hétéroazéotrope susdit de ce mélange et avec maintien d'un mélange exempt d'eau, contenant du chlorure de zinc et le diluant considéré, dans lequel on met en oeuvre comme diluant aprotique polaire un nitrile aliphatique, oléifiniquement insaturé, sélectionné à partir du groupe constitué de nitrile 2-cis-penténique, nitrile 2-trans-penténique, nitrile 3-cis-penténique, nitrile 3-trans-penténique, nitrile 4-penténique, nitrile E-2-méthyl-2-buténique, nitrile Z-2-méthyl-2-buténique, nitrile 2-méthyl-3-buténique ou leurs mélanges.

2. Procédé selon la revendication 1, dans lequel le diluant peut former un azéotrope ou un hétéroazéotrope avec de l'eau dans les conditions de distillation.

3. Procédé selon les revendications 1 à 2, dans lequel le mélange contenant de l'eau et du chlorure de zinc présente un pH inférieur à 7.

4. Procédé selon les revendications 1 à 3, dans lequel le mélange contenant de l'eau et du chlorure de zinc présente un pH compris entre 0 et 7 maximum.

5. Procédé selon les revendications 1 à 4, dans lequel le mélange contenant de l'eau et du chlorure de zinc, est additionné d'un acide.

6. Procédé selon la revendication 5, dans lequel l'acide mis en oeuvre est de l'acide chlorhydrique (HCl) .
